**Europäisches Patentamt**

**European Patent Office**

**Office européen des brevets**

(19)

(11) Publication number: **0 499 983 A1**

# EUROPEAN PATENT APPLICATION

(12)

(21) Application number: **92102517.7**

(22) Date of filing: **14.02.92**

(51) Int. Cl.⁵: **A61K 31/44**

(30) Priority: **15.02.91 SU 4911892**

(43) Date of publication of application:
**26.08.92 Bulletin 92/35**

(84) Designated Contracting States:
**BE DE DK ES FR GB IT SE**

(71) Applicant: **INSTITUTE OF ORGANIC SYNTHESIS OF THE LATVIAN ACADEMY OF SCIENCES**
**Aizkraukles 21**
**Riga-6(SU)**

(72) Inventor: **Duburs, Gunars J.**
**Ieriku 43-2**
**226059-Riga(SU)**
Inventor: **Bisenieks, Egils A.**
**Talavas Gatve 11-13**
**226029-Riga(SU)**
Inventor: **Germane, Skaidrite K.**
**Zala 3-16a**
**226010-Riga(SU)**
Inventor: **Klusa, Vija J.**
**Bruninieku 84-11**
**226009-Riga(SU)**
Inventor: **Bleidelis, Edite J.**
**Auces 9-1**
**226048-Riga(SU)**
Inventor: **Misane, Ilga L.**
**Valdemara 94-55**
**226013-Riga(SU)**

(74) Representative: **von Füner, Alexander, Dr. et al**
**Patentanwälte v. Füner, Ebbinghaus, Finck**
**Mariahilfplatz 2 & 3**
**W-8000 München 90(DE)**

(54) **Nootropic drug.**

(57) A nootropic drug is provided and a method for its use in the treatment of cognitive functions in warm-blooded animals. The active component of the drug is 2,6-dimethyl-3,5-bis[(2'-propoxy-ethoxycarbonyl]-4-(2''-difluorophenoxyphenyl)-1,4-dihydropyridine.

EP 0 499 983 A1

The invention relates to a nootropic drug. In one aspect, the invention relates to a nootropic drug and its use in the treatment of warm-blooded animals to improve cognitive functions, particularly those functions which have been impaired by stroke, cerebral trauma or neurodegenerative processes.

Recently, more and more attention is being paid to the search for new psychotropic - nootropic drugs activating the brain integrative functions, improving cognitive processes, i.e., thinking, attention, learning and memory (Mashkovsky, M.D., Roshchina, L.F., Poletayeva, A., Farmakol. Toxikol., 1977, No. 6, pgs. 676-683, Russia). An increasing interest in the drugs of this type is caused by augmentation of stress factors damaging neuronal, particularly membrane, communicative functions. Similarly, memory disorder is also induced by alcohol hypoxy senescence and the like.

Pyracetam is a traditionally used representative of a group of nootropic drugs, which positively affects metabolic processes in the brain and cerebral blood circulation (Mashkovsky, M.D., Roshchina, L.F., Poletayeva, A., Farmakol. Toxikol., 1977, No. 6, pgs. 676-683, Russia). Pyracetam is of a low toxicity, though in the experiments on animals to achieve its nootropic activity, the drug should be administered at very high doses, starting with 100-200 mg/kg. In recent years, according to the concept on the role of the cholinergic system in realization of memory processes, research in the memory improving drugs is directed to the substances of the tacrine type which are capable of blocking acetyle cholinesterase activity. The activity of such preparations, for example, amyridine, was detected within 0.1-0.2 mg/kg, and their toxicity is comparatively high - $LD^{50}$ = 54 mg/kg (Burov, V.Yu., Rokabidze, T.N., Portnov, Yu.N., Sukhanova, S.A., Peganov, E.M., Shapovalova, L.M., Zabrodnyaya, V.G., Egoricheva, L.A., Abstr. All-Union Conference "Estimation of Pharmacological Activity of Chemicals: Principles and Approaches", Part I., Moscow, 1989, pgs. 49-50, Russia). Due to peripheral cholinomimetic action, the drugs with this mechanism of action can induce a series of side effects (hypersalivation, bradycardia etc.).

Our goal was to develop a nootropic agent possessing a higher activity and lower toxicity as compared with the known drugs.

The goal was achieved by using a compound of the 1,4-dihydropyridineseries-2,6-dimethyl-3,5-bis[(2'-propoxy)ethoxycarbonyl)-4(2''-difluorophenoxyphenyl)-1,4-dihydropyridine (I). This compound was previously known to possess hypotensive and coronarodilating activity and also reveals cerebrovasodilating action (U.S.-Patent No. 4,845,109, C 07 D 211/86, A 61 K 31/455, 1989 and Bleidelis, E.J., Germane, S.K., Veveris, M.M., et al. In: Actual Problems of Pharmacology. Abstr., Kaunas, 1989, pg. 8, Russia). For the first time in the series of dihydropyridine compounds, it was detected that at very low doses ($\mu$g) they cause a considerable and long-term effect on learning acquisition and memory processes.

Figure 1 is a bar graph depicting the effect of Compound I (identified as IOS-1.1212) and Nimodipine on memory function.

Figure 2 shows bar graphs depicting the influence of Compound I and Nimodipine on amnesia induced by electro-convulsive shock.

Figure 3 shows bar graphs depicting the influence of Compound I and Nimodipine on Amnesia induced by Scopolamine.

Experimental studies of nootropic properties of Compound I were performed on Icr:Ic1, BALB/C mice and albine outbred male rats. The action of Compound I was compared with known 1,4-dihydropyridine derivatives - Nimodipine (II), 2,6-dimethyl-3-isopropoxycarbonyl-5-methoxycarbonyl-1,4-dihydropyridine and Nicardipine (III, YC-93, 2,6-dimethyl-3-methoxycarbonyl-5-(N-benzyl-N-methyl-amino)ethoxycarbonyl-4-(m-nitrophenyl)-1,4-dihydropyridine) and the reference nootropic drug - Pyracetam (IV, 2-oxo-1-pyrrolidinylacetate).

The substances under studies as a water suspension prepared with Tween-80 were administered intraperitoneally or orally in different periods before testing. Distilled water with addition of the respective concentration of Tween-80 was used as the pharmaceutically acceptable carrier, and was injected or administered into the stomach to control animals in the same periods of time and in the same volume as the substances under study.

Nootropic properties of Compound I were assessed according to the following tests:

1. Effect on normobaric hypoxic hypoxia was induced by placing every animal into a separate 220 $m^3$ hermetically sealed chamber without $CO_2$ absorption, the life span of every animal in the hermetic chamber being registered.

2. Effect on memory and antiamnestic activity.

The effect on memory was studied on a model of passive avoidance response (PAR). The time of mice stay in the experimental two-compartment chamber made up 180 sec., after which, in the dark compartment, the animals were subjected to inescapable foot shock stimulation through the chamber floor. The degree of PAR retention was assessed in the following 24 hours. The number of animals was counted who entered the dark compartment and the difference between entrance latency into the dark compartment after 24 hours and the first entrance into the dark compartment (t). Retro-grade amnesia was induced by maximal corneal electroshock (during 10-15 s) just after the learning session.

The effect of Compound I on the three processes of memory formation was assessed in these experiments:

1) On learning acquisition and PAR retention - the substance was administered 60 min. before the test;

2) On memory consolidation - Compound I was administered immediately after the learning trial;

3) On memory retrieval - Compound I was administered 1 hr. before the retention test;

4) PAR test with electroshock-induced amnesia was performed at I repeated (7 days) administration at various doses; and

5) As an amnestic agent, there was also used the M-cholinolytic agent Scopolamine which was given at a dose of 5 mg/kg 1 hour before the learning test.

3. Effect on acquisition, formation and retention of conditioned avoidance response (CAR).

Rats during the experiments were kept under standard vivarium conditions; 7 animals in a box with water and food ad libitum.

Effects of the drugs on the learning process was estimated in automatic device "Ugo Basile" with written registration of the animals' corresponding to response parameters. Programming and recording devices of "Ugo Basile" were in the light room, and two (2) two-compartment boxes with animals - in an acoustically isolated dark room. Each animal before learning, underwent an adaptation period, i.e. for 10 min. was placed into a box. Formation of CAR was performed in a two-compartment box supplied with a visual stimulus (light) serving as a conditioned stimulus. 3 sec. after switching of the conditioned stimulus, an animal received a nonconditioned reinforcement - a shock with electric current (voltage up to 150 v., frequency 50 Hz., duration 2 Sec.) through the grid floor. Every animal was subjected daily to a learning session consisting of 20 combinations of conditioned and nonconditioned stimuli. Criteria for learning performance served:

1) correct (80-100%) responses to switching of conditioned stimuli,

2) duration of latent period to stimuli.

Animals were trained in CAR conditions for 12 days. The substances under study were ip or orally administered 1 hr. daily before the learning trial. Inhibition of CAR extinction was tested (only upon switching of conditioned stimuli) on days 5, 10 and 20 after interruption of administration of the substances.

All the experimental data was statistically processed calculating mean arithmetical values and standard error of these mean values (M±MO). Student's t criterion was used to evaluate the significance of difference between mean values. The differences were considered to be significant at P<0.05.

The results concerning a comparative study of Compounds (I), Nimodipine (II), Nicardipine (III) and Pyracetam (IV) are given in Tables 1-5.

EP 0 499 983 A1

<u>Table 1</u>

Antihypoxic activities (life span, min(%) of compounds IOS-1.1212 (I), Nimodipine (II), Nicardipine (III) and Pyracetam (IV) at intraperitoneal and oral administrations in tests on Icr:Icl and BALB/c male mice weighing 19-23 g (n = 6, t° = 21-25°C)

| Dose, mg/kg | Amimal | Admi-nistr. route | IOS-1.1212 (I) | | | | Nimodipine (II) | |
|---|---|---|---|---|---|---|---|---|
| | | | 30 min | | 60 | | 60 | |
| | | | M+MO | % | M+MO | % | M+MO | % |
| 1 | 2 | 3 | 4 | 5 | 6 | 7 | 8 | 9 |
| | | | | | <u>C o n t r o l : 21,3 + 0,5 (100%)</u> | | | |
| 0,005 | BALB/c | ip | 30,0±2,5* (140,8) | | – | – | 19,6±1,5 (92,0) | |
| 0,05 | " | " | 32,9±3,8* (154,5) | | – | – | 19,6±2,1 (92,0) | |
| 0,5 | " | " | 33,0±2,5* (154,9) | | – | – | 22,8±2,0 (107,1) | |
| 1,5 | " | " | – | – | 28,3±2,4* (132,2) | | 27,1±1,9*(127,3) | |
| 5,0 | " | " | 38,1±1,9* (178,9) | | – | – | 27,5±1,7*(129,1) | |
| 0,1 | " | po | – | – | 25,5±3,0 (119,7) | | – | – |
| 1,0 | " | " | – | – | 30,1±3,6* (147,3) | | – | – |
| 5,0 | " | " | – | – | 43,5±4,9* (204,3) | | – | – |
| 0,05 | Icr:Icl | ip | 27,9±3,0* (139,9) | | 47,6±2,7* (203,5) | | 23,7±1,3 (111,3) | |
| 0,5 | " | " | 29,0±2,4* (136,4) | | 48,8±3,4* (229,1) | | 24,5±1,5 (115,0) | |
| 1,5 | " | " | 32,8±6,2* (154,0) | | – | – | 24,8±1,2 (116,4) | |
| 5,0 | " | " | 34,8±6,2* (163,8) | | 52,8±2,5* (248,0) | | 26,5±1,7*(132,5) | |
| 25,0 | BALB/c | | – | – | – | – | – | – |
| 100,0 | " | " | – | – | – | – | – | – |
| 250,0 | " | " | – | – | – | – | – | – |

EP 0 499 983 A1

Table 1 (cont'd)

| Nicardipine (III) | | | | Pyracetam (IV) | |
| --- | --- | --- | --- | --- | --- |
| 30 | | 60 | | 60 | |
| M+MO | % | M+MO | % | M+MO | % |
| 10 | 11 | 12 | 13 | 14 | 15 |
| Control : 21,3+0,5 (100%) | | | | | |
| – | – | $19,2\pm3,0$ | (90,0) | – | – |
| $25,1\pm4,1$ | (117,8) | $22,3\pm1,7$ | (104,7) | $18,5\pm1,9$ | (86,9) |
| $33,6\pm4,5*$ | (157,7) | $23,9\pm2,8$ | (112,2) | $20,7\pm1,5$ | (97,2) |
| – | – | $28,9\pm4,0*$ | (135,7) | – | – |
| $32,9\pm3,5*$ | (154,5) | – | – | $27,7\pm3,5$ | (130,0) |
| – | – | $30,2\pm3,5$ | (141,8) | – | – |
| – | – | $29,9\pm4,3$ | (140,3) | – | – |
| – | – | $39,0\pm4,7*$ | (183,1) | – | – |
| – | – | $26,7\pm2,9$ | (125,3) | – | – |
| – | – | $30,8\pm1,4*$ | (144,6) | – | – |
| – | – | $31,7\pm2,3*$ | (148,8) | – | – |
| – | – | $29,3\pm1,9$ | (146,3) | – | – |
| – | – | – | – | $30,5\pm7,1$ | (142,9) |
| – | – | – | – | $28,9\pm3,5$ | (135,7) |
| – | – | – | – | $32,7\pm3,8*$ | (153,5) |

* Differences against the control are statistically significant within $p \leq 0,05-0,001$

## Table 2

Comparative data on the capabilities of compound IOS-1.1212 (I), Nimodipine (II), Nicardipine (III) and Pyracetam (IV) to prevent PAR disturbance upon the action of maximal electroshock as an amnesic factor. The tests were performed on BALB/c male mice weighing 19-23 g (n = 10, t° = 22-23°C)

| Drug | Dose, mg/kg | PAR latency, s | | | | Antiamnestic activity, % | |
| | | 1 hr | | 3 hrs | | 1 hr | 3 hrs |
| | | M+MO | p | M+MO | p | | |
|------|------|------|------|------|------|------|------|
| | | C o n t r o l : 13,0+8,9 (100%) | | | | | |
| IOS-1.1212 | 0,05 | 48,3±15,6 | <0,05 | - | - | 66,6 | - |
| | 0,5 | 49,5±13,5 | <0,02 | - | - | 75,6 | - |
| | 1,0 | 68,3±16,0 | <0,01 | - | - | 85,6 | - |
| | 5,0 | 122,1±12,1 | <0,001 | 98,5±12,1 | <0,05 | 100,0 | 80 |
| Nimodipine | 0,05 | 6,6± 5,0 | >0,5 | - | - | 16,6 | - |
| | 0,5 | 29,2+19,2 | >0,5 | - | - | 50,0 | - |
| | 1,0 | 50,0±12,2 | <0,01 | - | - | 66,7 | - |
| | 5,0 | 56,0±15,2 | <0,05 | 46,5±16,2 | >0,1 | 50,0 | 70 |
| Nicardipine | 0,05 | 30,0± 1,8 | >0,5 | - | - | 30,0 | - |
| | 0,5 | 18,5±10,4 | >0,5 | - | - | 30,0 | - |
| | 1,0 | 25,5±13,7 | >0,1 | - | - | 55,6 | - |
| | 5,0 | 14,0± 4,6 | >0,5 | 19,0± 7,1 | >0,5 | 40,0 | 50 |
| Pyracetam | 5,0 | 24,5± 6,9 | >0,25 | - | - | 16,6 | - |
| | 50,0 | 30,8±14,1 | >0,25 | - | - | 33,3 | - |
| | 100,0 | 58,6±20,8 | <0,05 | - | - | 66,6 | - |
| | 250,0 | 85,2±17,2 | <0,05 | - | - | 83,3 | - |

6

EP 0 499 983 A1

## Table 3

Effect of IOS-1.1212 on the conditioned avoidance response performance (CAR)

in the tests on albino outbred male rats weighing 200±10 g.

The substance under study was ip and orally (**) administered during 12 days

1 hr before learning trial (n = 8, z = 21-23°C)

| Days of inves-tigat. | CAR latency, (M+MO) s (%) | | | |
|---|---|---|---|---|
| | Control | Dose of IOS-1.1212, mg/kg | | |
| | 0 | 0,005 | 0,05 | 0,05** |
| 1 | 78,7± 6,9 | 72,2± 9,0 (91,7) | 77,4±12,1 (98,4) | 79,7± 7,8 (101,1) |
| 2 | 77,0± 6,3 | 54,0±13,4 (70,1) | 55,3±13,4 (71,8) | 65,4± 9,9 ( 84,9) |
| 3 | 68,9± 6,4 | 46,8±11,3 (67,9) | 51,7±12,5 (75,0) | 60,3± 7,8 ( 87,4) |
| 4 | 61,2± 6,2 | 47,0± 1,8 (76,7) | 45,8± 7,9 (74,9) | 54,8± 5,2 ( 89,5) |
| 5 | 59,7± 6,1 | 41,6± 2,9 (69,6) | 43,7± 3,8 (73,2) | 52,0± 7,6 ( 87,0) |
| 6 | 52,5± 7,4 | 40,2± 2,9 (76,5) | 39,8± 3,6*(75,8) | 49,5± 6,7 ( 94,3) |
| 7 | 52,9± 4,7 | 40,3± 2,5 (76,1) | 38,0± 2,9*(71,7) | 42,1± 4,0 ( 79,5) |
| 8 | 53,2± 6,0 | 41,3± 2,8 (77,6) | 35,1± 3,4*(65,9) | 40,8± 3,3*( 77,0) |
| 9 | 53,0± 4,7 | 40,6± 2,4 (76,6) | 34,1± 3,9*(64,3) | 39,7± 2,4*( 75,0) |
| 10 | 49,2± 4,8 | 41,3± 2,4 (83,9) | 34,6± 5,3*(70,3) | 38,2± 2,9*( 77,7) |
| 11 | 49,6± 4,6 | 40,9± 1,6*(82,4) | 32,5± 5,5*(65,6) | 36,1± 2,5*( 72,9) |
| 12 | 49,1± 4,6 | 38,6± 3,7*(78,6) | 34,4± 2,4*(70,1) | 37,5± 2,3*( 76,4) |
| 17 (5) | 69,7± 4,5 | 54,3± 5,5*(77,9) | 42,9± 6,3*(61,5) | 35,4± 6,7*( 50,6) |
| 22 (10) | 80,2± 9,1 | 51,5± 3,0*(69,2) | 40,8± 2,5*(56,9) | 48,8± 5,1*( 60,8) |
| 32 (20) | 89,9± 4,5 | 55,9±12,5*(62,2) | 50,8±12,5*(56,3) | 45,5± 8,0*( 50,6) |

Table 3 (cont'd)

| Days of investigat. | CAR latency, (M+MO) s (%) Dose of IOS-1.1212, mg/kg | |
| --- | --- | --- |
| | 0,5 | 1,5 |
| 1 | 72,0±14,3 (91,2) | 75,2±11,4 (95,5) |
| 2 | 61,5±11,9 (79,8) | 95,3±11,3 (123,6) |
| 3 | 60,0±10,0 (87,0) | 85,1±10,5 (123,5) |
| 4 | 60,9±10,9 (99,5) | 86,2±14,0 (140,8) |
| 5 | 51,1± 8,7 (86,2) | 68,5±11,7 (114,7) |
| 6 | 50,8± 9,1 (77,7) | 67,9±12,4 (129,3) |
| 7 | 54,9±12,3 (103,7) | 71,3±15,8* (134,7) |
| 8 | 54,6±13,3 (102,6) | 68,9±14,2 (129,5) |
| 9 | 55,5±10,3 (104,7) | 79,6±15,4* (150,2) |
| 10 | 54,8± 2,3 (111,4) | 76,8±14,5* (156,2) |
| 11 | 46,4±10,6 (93,5) | 56,1±13,5 (113,1) |
| 12 | 43,6± 8,4 (88,7) | 76,8±15,5* (156,4) |
| 17 (5) | 62,9±11,4 (90,2) | 96,8±13,6 (138,9) |
| 22 (10) | 87,1±10,5 (108,6) | 96,0±16,9 (119,7) |
| 32 (20) | 77,2±11,4 (85,8) | 109,0±19,4 (121,2) |

* Differences against the control are statistically significant at $p \leq 0,05$

EP 0 499 983 A1

## Table 4

Effects of Nimodipine (II), Nicardipine (III) and Pyracetam (IV) on conditioned avoidance response performance (CAR) in tests on albino outbred male rats weighing $200\pm15$ g. The compounds under study were ip administered during 12 days 1 hr before learning trial (n = 8, t = 21-23°C) (dose of Nimodipine and Nicardipine 0.05 mg/kg, that of Pyracetam 100 mg/kg)

| Days of inves- tigat. | CAR latency (M+MO) s (%) | | | | | |
|---|---|---|---|---|---|---|
| | Control | Nimodipine | (II) | Nicardipine (III) | Pyracetam | (IV) |
| 1 | 78,7± 6,9 | 72,9± 9,6 | (92,6) | 76,6± 9,5 (97,4) | 82,7± 6,0 | (105,1) |
| 2 | 77,0± 6,3 | 66,2± 8,5 | (85,9) | 62,1±14,2 (80,6) | 72,8± 4,1 | (94,5) |
| 3 | 68,9± 6,4 | 60,9± 9,2 | (88,4) | 51,5± 8,4 (74,8) | 60,4± 5,6 | (87,7) |
| 4 | 61,2± 6,2 | 54,7± 7,2 | (89,3) | 41,0± 4,2 (66,9) | 58,3± 4,4 | (95,2) |
| 5 | 59,7± 6,1 | 45,8± 4,3 | (76,6) | 43,6± 5,0 (72,9) | 56,6± 6,4 | (94,8) |
| 6 | 52,5± 7,4 | 40,3± 3,5 | (77,3) | 33,7± 4,8* (64,3) | 53,3± 6,2 | (100,7) |
| 7 | 52,9± 4,7 | 41,0± 5,3 | (77,4) | 37,1± 4,6* (70,2) | 50,3± 7,7 | (95,1) |
| 8 | 53,2± 6,0 | 37,9± 3,0* | (71,2) | 45,4± 6,0 (85,2) | 48,5± 7,5 | (91,1) |
| 9 | 53,0± 4,7 | 38,0± 4,8* | (71,6) | 45,6± 5,5 (86,0) | 46,4± 6,7 | (87,5) |
| 10 | 49,2± 4,8 | 38,2± 4,7* | (77,5) | 37,5± 4,0 (76,2) | 53,9± 6,8 | (109,5) |
| 11 | 49,6± 4,6 | 34,5± 4,6* | (69,5) | 39,2± 4,1 (79,0) | 49,9± 7,7 | (100,6) |
| 12 | 49,1± 4,6 | 30,0± 2,8* | (60,4) | 38,7± 7,9 (78,8) | 48,8± 6,9 | (99,4) |
| 17 (5) | 69,7± 4,5 | 52,1±11,3 | (74,8) | 57,8±15,4 (82,9) | 54,8± 8,9 | (78,6) |
| 22 (10) | 80,2± 9,1 | 60,1±14,3 | (74,9) | 59,9±13,4 (74,7) | 70,5±17,4 | (87,9) |
| 32 (20) | 89,9± 4,5 | 60,1± 9,1 | (66,6) | 71,8±14,8 (79,8) | 78,2± 9,9 | (86,9) |

* Differences against the control are statistically significant at $p \le 0.05$

EP 0 499 983 A1

Table 1 shows that Compound I possesses a higher anti-hypotic activity than the compounds compared. The action of Compound I is manifested already after 30 min. and reaches maximum following 1 hr. Unlike the compared compounds statistically, antihypoxic effect upon the action of Compound I reveals at very low doses - 0.005-0.05 mg/kg and with increase in its doses, the acitivity enhances. Compound I also manifests a high activity in formation of PAR and possesses a pronounced

## Table 5

The number of correct responses in CAR tests on male rats at intraperitoneal and oral (**) administrations of the compounds under study

| Days investi-gations | Control | IOS-1.1212, M+MO | | | | Nimodipine | | Nicardipine |
|---|---|---|---|---|---|---|---|---|
| | 0 (Doses) | 0,005 | 0,05 | 0,05** | 0,5 | 1,5 | 0,05 | 0,05 |
| 1 | 3,0±1,2 | 5,0±1,0 | 8,1±2,3 | 5,1±1,6 | 7,5±2,8 | 4,2±1,9 | 6,4±1,6 | 5,6±3,2 |
| 2 | 7,4±1,3 | 10,0±1,6 | 12,8±1,6 | 8,9±2,1 | 9,0±2,2 | 3,4±1,1 | 9,9±2,1 | 9,8±3,6 |
| 3 | 10,8±1,4 | 10,5±2,5 | 15,0±1,2 | 9,3±2,1 | 9,1±2,1 | 4,1±1,2 | 12,5±1,8 | 12,8±2,1 |
| 4 | 12,5±1,1 | 10,7±1,2 | 13,6±1,8 | 9,7±2,2 | 11,2±2,1 | 5,7±2,2 | 14,3±1,6 | 14,4±2,4 |
| 5 | 13,2±1,4 | 15,1±0,8 | 15,2±1,7 | 11,9±1,8 | 12,7±2,4 | 9,2±2,0 | 14,4±1,1 | 13,8±2,3 |
| 6 | 13,6±1,1 | 15,8±0,7 | 17,3±1,3* | 14,5±2,2 | 13,5±2,3 | 10,2±2,1 | 15,6±1,0 | 15,5±2,2 |
| 7 | 14,4±1,1 | 15,5±0,9 | 17,0±0,9* | 13,6±1,6 | 13,7±2,1 | 10,2±1,8 | 15,2±2,4 | 16,8±1,9 |
| 8 | 15,5±1,0 | 14,8±0,9 | 15,0±1,2 | 15,2±1,6 | 13,7±1,2 | 10,2±2,0 | 15,7±1,9 | 14,5±2,8 |
| 9 | 15,3±0,8 | 16,5±0,8 | 17,0±0,8 | 15,0±1,4 | 13,5±2,4 | 9,0±2,1* | 15,6±1,8 | 15,4±1,9 |
| 10 | 14,5±1,0 | 16,8±1,1 | 15,5±1,8 | 15,2±1,1 | 14,3±2,3 | 8,7±1,9* | 16,0±0,8 | 15,3±1,0 |
| 11 | 14,7±0,8 | 18,1±1,0* | 17,3±1,3* | 17,7±0,5* | 15,6±2,5 | 9,0±1,3* | 16,9±0,7* | 16,0±1,8 |
| 12 | 14,8±1,0 | 17,5±1,3* | 17,3±1,7* | 17,7±0,5* | 15,8±1,7 | 8,6±2,2* | 17,7±0,8* | 14,3±2,8 |
| 17 (5) | 11,0±1,4 | 17,4±1,2* | 16,3±1,3* | 17,2±1,6* | 15,2±2,0 | 10,4±2,2* | 13,0±1,5 | 13,2±1,6 |
| 22 (10) | 8,8±1,5 | 14,5±0,6* | 15,8±0,8* | 15,7±1,2* | 7,6±2,1 | 7,7±2,3 | 12,4± 1,6 | 13,1±2,4 |
| 32 (20) | 7,7±2,0 | 11,0±0,6 | 13,3±1,5* | 15,9±1,5 | 8,4±2,4 | 4,7±2,3 | 10,2±1,7 | 9,3±2,4 |

* Differences are statistically significant against the control at $p \leq 0.05$

antiamnestic action (66.6-100% prevents retrograde amnesia), (Table 2). At the same time, simultaneously studied compounds have a rather less activity. So, Nimodipine has statistically significant parameters at 20 times higher doses and only in 66.7% prevents retrograde amnesia, and Nicardipine possesses no marked activity at all according to the given tests, and Pyracetam only at a dose of 100 mg/kg has some slight action.

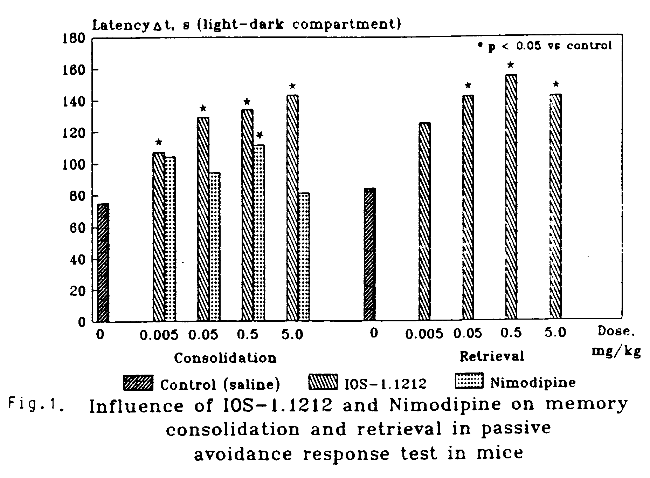

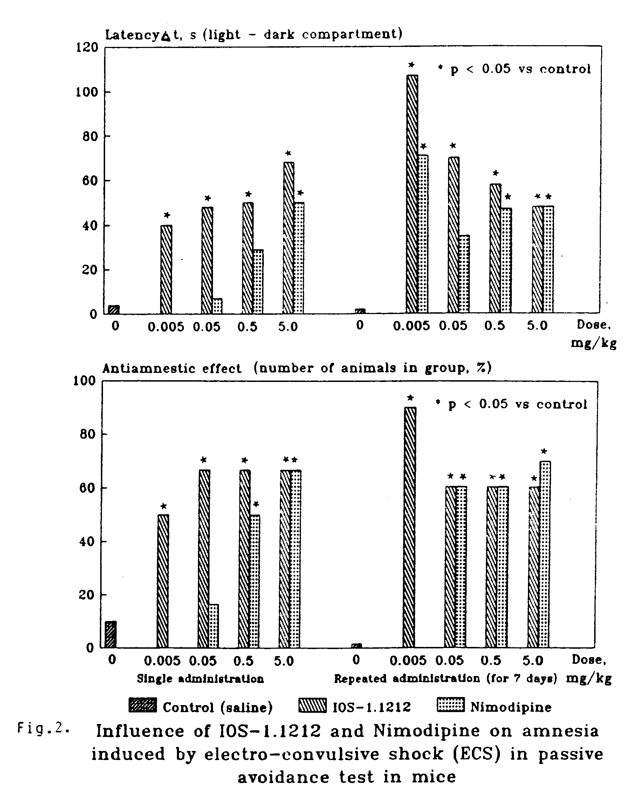

The data presented in Fig. 1 shows that Compound I is a strong agent affecting the processes of memory consolidation and retrieval. At repeated (7 days) administration, Compound I highly enhances learning potency (Fig. 2a [upper part]), especially at low doses (0.005 and 0.05 mg/kg) and antiamnestic activity (Fig. 2b, [lower part]). Nimodipine causes similar effects only at doses of 0.5 and 5 mg/kg.

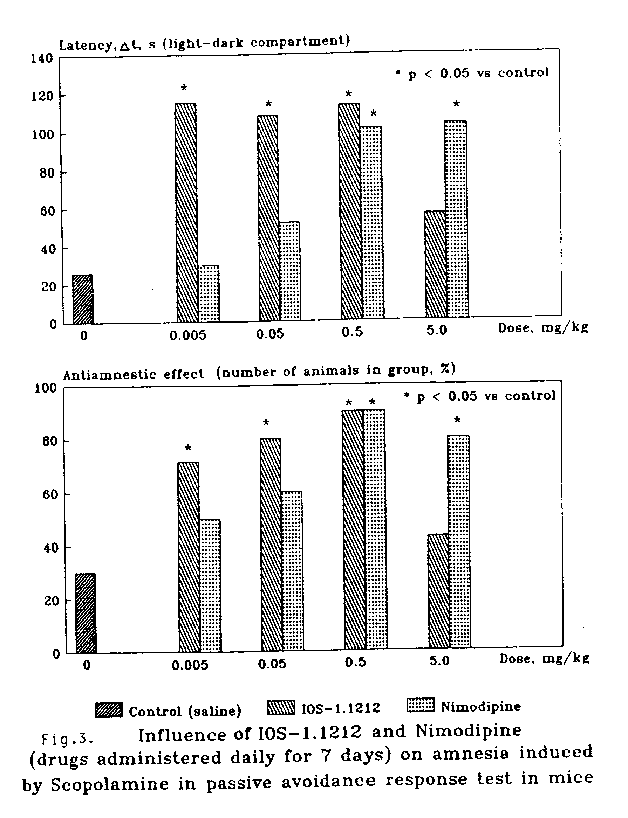

A high antiamnestic activity was also found in the PAR test with Scopolamine amnesia (Fig. 3b).

With respect to a conditioned avoidance response (CAR), it was elucidated that the action of Compound I highly depends on the dose administered (Tables 3-5). So, Compound I at low doses (0.005-0.05 mg/kg) stimulates CAR formation, and at higher doses (1.5 mg/kg) on the contrary, retards it (Tables 3-5). Simultaneously, examined reference substances (Nicardipine, Nimodipine at a dose of 0.05 mg/kg, Pyracetam at a dose of 100 mg/kg), have markedly lower activity (Table 4) according to every test than Compound I.

A very important advantage of Compound I is its long-lasting memory improving effect after interruption of its administration for 12 days at doses of 0.005-0.05 mg/kg (Tables 3-5).

Thus, this data demonstrates that 2,6-dimethyl-3,5-bis[(2'-propoxy)ethoxycarbonyl]-4-(2''-difluorophenoxyphenyl)-1,4-dihydropyridine possesses a considerably more pronounced nootropic activity which is manifested at its administration at very low doses.

Taking into account a marked antihypoxic activity, activating action on PAR and CAR formation, as well as its high antiamnestic effect, the proposed 1,4-dihydropyridine derivative may find its application in medicine as a nootropic agent for long-term improvement of learning acquisition, memory formation, disturbed by various factors - hypoxy, stroke, cerebral trauma, senescence, neurodegenerative processes, Alzheimer's diseases, AIDS, etc.

**Claims**

1. Use of 2,6-dimethyl-3,5-bis[(2'propoxy)ethoxycarbonyl]-4-(2''-difluorophenoxyphenyl)-1,4-dihydropyridine for the preparation (manufacturing) of a medicine useful for the improvement of cognitive functions (attention, learning, memory) impaired by stroke, cerebral trauma, neurodegenerative processes (Alzheimer's disease, AIDS), senile dementia.

Fig.1. Influence of IOS-1.1212 and Nimodipine on memory
consolidation and retrieval in passive
avoidance response test in mice

Latency△t, s (light – dark compartment)

* p < 0.05 vs control

Dose, mg/kg

Antiamnestic effect (number of animals in group, %)

* p < 0.05 vs control

Dose,
Single administration          Repeated administration (for 7 days)   mg/kg

Control (saline)        IOS-1.1212        Nimodipine

Fig.2.    Influence of IOS-1.1212 and Nimodipine on amnesia
induced by electro–convulsive shock (ECS) in passive
avoidance test in mice

13

Fig.3.  Influence of IOS-1.1212 and Nimodipine
(drugs administered daily for 7 days) on amnesia induced
by Scopolamine in passive avoidance response test in mice

European Patent Office

**PARTIAL EUROPEAN SEARCH REPORT** Application Number

which under Rule 45 of the European Patent Convention shall be considered, for the purposes of subsequent proceedings, as the European search report

EP 92 10 2517

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | ARZNEIMITTEL FORSCHUNG, vol. 39, no. 10, 1989, pages 1185-1189; G.J. DUBUR et al.: "Synthesis and selective vasodilating properties of esters of 2,6-dimethyl-4-(2-difluoromethoxyphenyl)-1,4-dihydro-pyridine-3,5-dicarboxylic acid" * Whole document * --- | 1 | A 61 K 31/44 |
| D,X | GB-A-2 180 532 (INST. ORGANICH. SINTEZA AKAD. NAUK LATVIISKOI) * Whole document * --- | 1 | |
| A | EP-A-0 311 053 (BANYU PHARMACEUTICAL CO.) * Abstract; page 19, lines 16-36 * --- | 1 | |
| A | EP-A-0 004 650 (BAYER AG) * Whole document * --- -/- | 1 | |

TECHNICAL FIELDS SEARCHED (Int. Cl.5)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

The compound mentioned in the claim 1 doesn't correspond to the compound described in the US patent no. 4 845 109 to which the application makes explicitly reference. The search has been however carried out for the compound defined in the US patent (ex.3).

| Place of search | Date of completion of the search | Examiner |
|---|---|---|
| THE HAGUE | 30-03-1992 | HOFF P.J.L. |

EPO FORM 1503 03.82 (P0407)

**PARTIAL EUROPEAN SEARCH REPORT**

| | DOCUMENTS CONSIDERED TO BE RELEVANT | | CLASSIFICATION OF THE APPLICATION (Int. Cl.5) |
|---|---|---|---|
| Category | Citation of document with indication, where appropriate, of relevant passages | Relevant to claim | |
| A | EP-A-0 253 173 (FUJISAWA PHARMACEUTICAL CO.) * Abstract; page 2, line 21 - page 3, line 30 * --- | 1 | |
| A | THE JAPANESE JOURNAL OF PHARMACOLOGY, vol. 38, no. 1, May 1985, pages 1-7; A. WAUQUIER et al.: ""Calcium entry blockers as cerebral protecting agents: comparative activity in tests of hypoxia and hyperexcitability" * Whole document * --- | 1 | |
| A | FR-A-2 545 354 (BAYER AG) * Whole document * --- | 1 | TECHNICAL FIELDS SEARCHED (Int. Cl.5) |
| A | EP-A-0 229 644 (WARNER-LAMBERT CO.) * Abstract; claims * --- | 1 | |
| A | WO-A-9 011 761 (THE CHILDREN'S MEDICAL CENTER CORP.) * Abstract; claims * ----- | 1 | |